# EUROPÄISCHE PATENTSCHRIFT

(11) **EP 1 970 059 B1**
(45) Veröffentlichungstag und Bekanntmachung des Hinweises auf die Patenterteilung: **25.11.2009**
(21) Anmeldenummer: 07005084.4
(22) Anmeldetag: 12.03.2007
(51) Int. Cl.: A61K 31/185, A61K 31/352, A61K 31/60, A61P 19/02, A61K 45/06

(54) **Arzneimittel mit Dobesilat-Calcium zur Behandlung und Prophylaxe von Sehnenerkrankungen**
Medication with Dobesilat-Calcium for treatment and prophylaxis of tendon disease
Médicament avec Dobesilate de Calcium destiné au traitement et à la prophylaxie des maladies des tendons

(43) Veröffentlichungstag der Anmeldung: 17.09.2008
(73) Patentinhaber: DOBAVET GmbH, 8806 Bäch (CH)
(72) Erfinder: Stuker, Gerhard, Dr., 8806 Bäch (CH); Fricker, Christian, 8165 Schlofflisdorf (CH)
(74) Vertreter: Vossius & Partner

(56) Entgegenhaltungen:
- WO-A-90/11759
- WO-A-95/08992
- SOSA M ET AL: "Metabolism of the calcium and bioavailability of the salts of most frequent use" BOLLETTINO CHIMICO FARMACEUTICO 2003 ITALY, Bd. 142, Nr. 1, 2003, Seiten 28-33, XP008082088 ISSN: 0006-6648
- STENLUND B: "Shoulder tendinitis and osteoarthrosis of the acromioclavicular joint and their relation to sports" BRITISH JOURNAL OF SPORTS MEDICINE 1993 UNITED KINGDOM, Bd. 27, Nr. 2, 1993, Seiten 125-130, XP008082133 ISSN: 0306-3674
- JUBB, KENNEDY UND PALMER?S: PATHOLOGY OF DOMESTIC ANIMALS, VOLUME 1, ELSEVIER 2007: 2007,
- GOODSHIP A.E. ET AL.: T: "the pathobiology and repair of tendon and ligament injury" VET. CLIN. NORTH. AM. EQUINE PRACT. 1994, 10, 323-349,
- KOBAYASHI A. ET AL.: "Morphological and histochemical analysis of a case of superficial digital flexon tendon injury in the horse" J. COMP. PATHOL. 1999, 120,, Seite 403,
- WEBBON P.M.: A POST MORTEM STUDY OF EQUINE DIGITAL FLEXOR TENDONS. EQUINE VET. J. 1977, 9, 61-67:
- JOHNSTON S.A.:: "Osteoarthritis: joint anatomy, physiology and pathobiology." VET. CLIN. NORTH. AM. SMALL ANIM. PRACT. 1997, 27, 699-723,

## Beschreibung

Die Erfindung betrifft Dobesilat-Calcium zur Behandlung und/oder Prophylaxe von Sehnenerkrankungen, bzw. ein Arzneimittel enthaltend Dobesilat-Calcium zur Behandlung und/oder Prophylaxe von Sehnenerkrankungen.
Ferner betrifft die Erfindung die Verwendung von Dobesilat-Calcium zur Herstellung eines Arzneimittels zur Behandlung und/oder Prophylaxe von Sehnenerkrankungen.
Die Erfindung betrifft des weiteren auch ein Verfahren zur Behandlung und Prophylaxe derartiger Sehnenerkrankungen, wobei eine wirksame Menge der vorstehend genannten Verbindung einem Säuger verabreicht wird.
Die Erfindung betrifft des weiteren die Verwendung von Dobesilat-Calcium in Kombination mit einem thrombozytenaggregationshemmenden Wirkstoff zur Herstellung eines Arzneimittels zur Behandlung und/oder Prophylaxe derartiger Sehnenerkrankungen. Die zu behandelnde Sehnenerkrankung kann eine Tendinitis darstellen oder auf degenerativen Prozessen beruhen.

Der bisherige Einsatz von Dobesilat-Calcium zur Behandlung und Prophylaxe von Knochen- und Gelenkserkrankungen wird in EP-0 670 721 B1 beschrieben In US-A-3 509 207 wird der Einsatz von Dobesilat-Calcium zum Stillen von Blutungen beschrieben.

WO 9 508 992 und WO 9 011 759 offenbaren Dobesilat-Calcium zur Behandlung von Gelenkerkrankungen.

Tendinitis ist eine Sehnenentzündung, welche im eigentlichen Sinne die entzündliche Veränderung des Sehnengewebes bezeichnet. Degenerative Veränderungen des betroffenen Gewebes, evtl. auch mit späteren Kalkeinlagerungen, sind häufig die Folge. Prinzipiell kann eine Tendinitis jede Sehne des Körpers betreffen. Da die Hauptursache eine mechanische Überlastung ist (Sport), tritt eine Tendinitis bevorzugt in bestimmten Körperregionen auf, so z.B. im Bereich der Schulter und des Schienbeins bzw. Fußes. Eine Tendinitis kann aber auch im Rahmen von entzündlichen rheumatischen Erkrankungen (insbesondere Reiter-Syndrom, Spondylitis ankylosans, Arthritis psoriatica) auftreten.

*Die drei häufigsten Formen einer Tendinitis beim Menschen sind:*
1. **Tendinitis** der Sehne des **Musculus tibialis anterior**
2. **Tendinitis** calcarea (meistens Schultergelenk betroffen)
3. **Tendinitis** der **Achillessehne**

Bisherige Therapiemethoden der häufig auftretenden Formen der Tendinitis
1. Die Therapie der Tendinitis der Sehne des Musculus tibialis anterior besteht in Ruhigstellung des Unterschenkels und Fußes, in schweren mit Gipsverband. Hilfreich sind auch Eisanwendungen und antiphlogistische Schmerzmittel. In hartnäckigen Fällen kommt die therapeutische Lokalanästhesie zum Einsatz und zwar in Form von wiederholten Infiltrationen um die Sehn e herum mit einem langwirkenden örtlichen Betäubungsmittel, zwischendurch auch mit Cortison-Zusatz.
2. Bei der Behandlung der Tendinitis calcarea wird anfänglich, in der akuten Phase, die Schulter ruhiggestellt (Armschlinge), später dann Krankengymnastik in Form von Pendelübungen, zunächst passiv, dann auch aktiv, durchgeführt. Hilfreich sind auch Kältepackungen. Medikamentös werden nichtsteroidale Antirheumatika verordnet. Wenn diese Maßnahmen nicht ausreichen, kommt die therapeutische Lokalanästhesie zum Einsatz und zwar in Form von wiederholten Infiltrationen mit einem langwirkenden örtlichen Betäubungsmittel, zwischendurch auch mit Cortison-Zusatz. Leider gibt es immer wieder Fälle, bei denen trotz adäquater Behandlung weiterhin eine schmerzhafte Tendinitis (chronische) besteht. Eine sehr hilfreiche Methode ist bei dieser Tendinitis die kontinuierliche Blockade des Plexus brachialis bei der durch einen Katheter mehrmals täglich, jeweils nach Abklingen der vorangegangenen Dosis, das örtliche Betäubungsmittel völlig schmerzlos nachgespritzt wird. Dass Lokalanästhetika auch entzündungshemmend wirken, ist zwischenzeitlich wissenschaftlich erwiesen.
3. Bei der Tendinitis der Achillessehne kommen physikalischen Therapieformen, kühlende Eispackungen, Ultraschall- und Gleichstromtherapie sowie die lontophorese zum Einsatz. Diese sind in der Lage die Problematik zwar etwas zu lindern, der Zeitfaktor bleibt aber bestehen. Oft macht ein Fersenkeil als Schuheinlage sehr viel Sinn bzw. eine entsprechende Bandage. Bei Einrissen der Sehne können lokal kortisonfreie Schmerzmittel aufgetragen werden. Ein Riss der Achillessehne muss nicht operativ behandelt werden, wenn die Enden nahe genug beieinander liegen und der Patient ein höheres Lebensalter hat. Von Injektionen mit Kortisonpräparaten wird abgeraten, da bei einem übersehenen Teilriss der Achillessehne ein durch Kristallisation des Kortison in der Sehne ein Totalabriss hervorgerufen werden könnte. Bei einer stark verkalkten Sehne (auch eine mögliche Ursache für die Tendinitis der Achillessehne) können vernarbte Teile der Achillessehne operativ entfernt werden. Des weiteren wird eine spezielle Schmerztherapie bei einer Tendinitis der Achillessehne angewendet. Eine erfolgreiche Behandlung muss zwangsläufig sowohl den Schmerz als auch die Entzündung betreffen. Dazu einet sich in vorzüglichem Maße die therapeutische Lokalanästhesie. Die lokale Infiltration mit einem Lokalanästhetikum ist allerdings relativ schmerzhaft und eignet sich deshalb kaum zur serienmäßigen, wiederholten Anwendung. Besser sind bei chronischer Tendinitis der Achillessehne wiederholte Blockaden des N. ischiadicus, in hartnäckigen Fällen optimal kontinuierlich mit Katheter.

Insbesondere betrifft die vorliegende Erfindung auch die Verwendung in der Tiermedizin, insbesondere beim Pferd.

In einer bevorzugten Ausführungsform liegt eine Tendinitis der oberflächlichen Beugungssehne beim Pferd vor. Auf Grund ihrer Häufigkeit ist die Tendinitis der oberflächlichen Beugesehne die am besten untersuchte Sehnenerkrankung beim Pferd. Bei der oberflächlichen Beugesehne des Pferdes handelt es sich um eine elastische Struktur, die unter maximaler Belastung an die Grenzen ihrer physiologischen Funktionalität stößt. Die biomechanischen und biochemischen Reaktionen der oberflächlichen Beugesehne auf Arbeit, Verletzung und die Heilungsvorgänge sind immer noch nicht abschließend bekannt. Neuere Ergebnisse wissenschaftlicher Untersuchungen liefern jedoch wertvolle Informationen. Offenbar reift das Gewebe der oberflächlichen Beugesehne früh in der Ontogenese aus. Ist die Entwicklung erst einmal abgeschlossen, so bestehen so gut wie keine Möglichkeiten der Anpassung des Sehnengewebes an außergewöhnliche Belastungen, etwa durch eine Erhöhung der Elastizität. Bei Belastung (Stress) der Sehne kommt es daher immer zu einer progressiv verlaufenden Gewebedegeneration. Eine fokale Abnahme von Zellen, die Degeneration von Kollagenfibrillen, eine selektive Zunahme der auf den Fibrillen lastenden Kräfte sowie Alterationen der nichtkollagenösen Gewebematrix zeigen sich hauptsächlich im zentralen, mittleren Bereich der Sehne auf Höhe des Metakarpus.

Die heute üblichen Behandlungsstrategien, der Tendinitis der oberflächlichen Beugungssehne beim Pferd, die darauf abzielen, ein Sportpferd soweit wiederherzustellen, dass es eine maximale Leistung erbringen kann, zeigen sehr unterschiedliche und im Gesamtergebnis unbefriedigende Ergebnisse. Moderne Rehabilitationsmaßnahmen sind, wenn sie mit regelmäßigen Ultraschallkontrolluntersuchungen gekoppelt werden, den chirurgischen Behandlungsmaßnahmen durchaus gleichwertig und auch kosteneffektiver. In neuerer Zeit hat sich das wissenschaftliche Interesse vor allem auf die pharmakologische Modulation der an den Kollagenstrukturen ablaufenden Heilungsprozesse konzentriert. Es wurden diverse Wachstumsfaktoren als potentielle therapeutische Mittel zur Unterstützung der Heilung bei Sehnenverletzungen erforscht.

Erfindungsgemäß konnte festgestellt werden, dass bei Sehnenerkrankungen wie der Tendinitis durch Behandlung mit Dobesilat-Calcium zu einer Gesundung des betroffenen Gewebes führt. Dies trifft insbesondere in der Humanmedizin für den Problemkreis der verschiedenen Formen der Achillessehnenentzündungen und in der Veterinärmedizin für die Tendinitis der oberflächlichen Beugungssehne des Pferdes zu.
Erfindungsgemäss konnte nachgewiesen werden, dass die erkrankten Sehnen durch die Behandlung ihre ursprüngliche Struktur wieder erlangen.

Die erfindungsgemäßen Arzneimittel können zusätzlich zu Dobesilat-Calcium thrombozytenaggregationshemmende Wirkstoffe wie Acetylsalicylsäure (ASS) und/oder Benzopyronverbindungen enthalten.

Die Erfindung umfasst die Verwendung von Dobesilat-Calcium der Formel

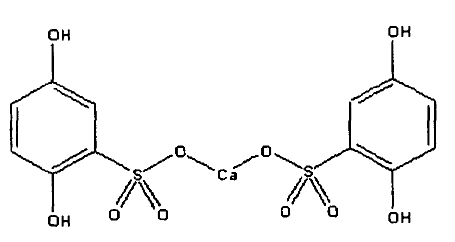

Die Herstellung dieser Verbindung wird in US-A-3 509 207 beschrieben.

In Kombination mit Dobesilat-Calcium sind für die erfindungsgemäße Verwendung sind folgende Benzopyrone geeignet:
1,2-Benzopyron-Derivate der allgemeinen Formel I 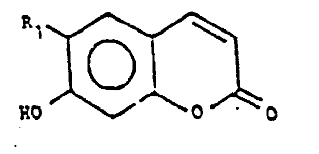 in der R₁ Wasserstoff, Halogen oder eine Hydroxy-, Sulfonyl-, Alkyl-, Hydroxyalkyl-, Acyloxy-, Alkoxy- oder Benzylgruppe oder ein Glycosidrest ist, sowie 1,4-Benzopyrone der allgemeinen Formel II 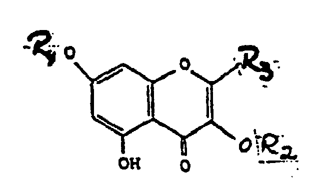 in der R₂ und R₄ unabhängig voneinander Wasserstoff, Halogen oder eine Hydroxy-, Sulfonyl-, Alkyl-, Hydroxyalkyl-, Acyloxy-, Alkoxy- oder Benzylgruppe oder ein Glycosidrest sind, und R₃ Wasserstoff oder einen Phenylrest der allgemeinen Formel 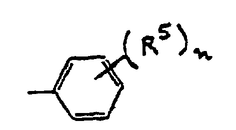 bedeutet, in der R⁵ ein Halogenatom, eine Hydroxy-, Sulfonyl-, Alkyl-, Hydroxyalkyl-, Acyloxy-, Alkoxygruppe ist und n Werte zwischen 0 bis 3 annehmen kann.

In Kombination mit Dobesilat-Calcium sind für die erfindungsgemäße Verwendung sind folgende Benzopyrone besonders bevorzugt: 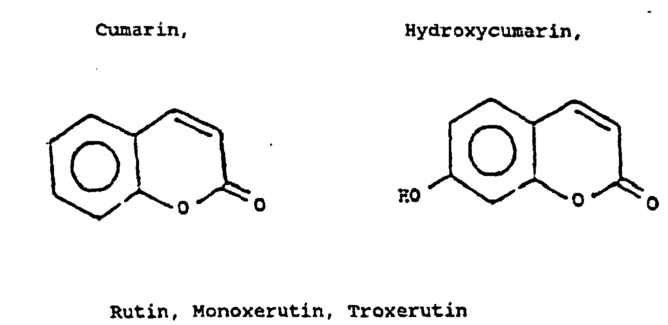 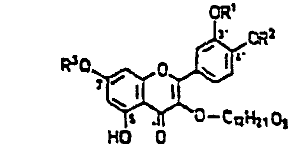 R¹ = R² = R³ = H; Rutein
R¹ = R² = H, R³ = (CH₂)₂-OH Monocerutin
R¹ = R² = R³ = (CH₂)₂-OH Troxerutin

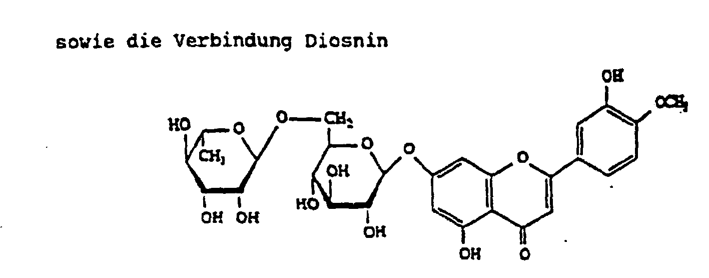

Die vorstehenden Verbindungen können in an sich bekannter Weise hergestellt werden, vgl. Beilstein III/IV, Bd. 18, S294ff. Des weiteren sind diese Verbindungen als Naturstoffe im Handel erhältlich.

In Kombination mit Dobesilat-Calcium ist für die erfindungsgemäße Verwendung Acetylsalicyclsäure besonders geeignet:

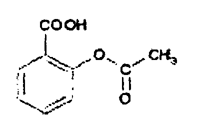

Die vorstehenden Verbindung kann in einer dem Fachmann bekannter Weise hergestellt werden. Des weiteren sind diese Verbindungen im Handel erhältlich.

Die vorliegende Erfindung stellt zudem auch Arzneimittel bereit die Dobesilat-Calcium, gegebenenfalls in Kombination mit einem weiteren erfindungsgemässen Wirkstoff, gegebenenfalls unter Beimischung von im Fachgebiet üblichen Hilfsstoffen und Exzipienten umfassen. Die erfindungsgemäßen Arzneimittel können nach üblichen dem Fachmann bekannten Verfahren und Techniken formuliert/hergestellt werden; wie z.B. in Remington's Pharmaceutical Sciences, 15 Auflage, Mack Publishing Co., New Jersey (1991) beschrieben.

Bevorzugt sind hierbei Darreichungsformen zur oralen, parenteralen (z.B. i..v., s.c., i.p., i.c., intrathekal) und lokalen (z. B. topisch, rectal, vaginal, buccal Applikation im Auge oder durch Inhalation) Applikation.
Somit können die erfindungsgemäßen Arzneimittel insbesondere als Tabletten (insbesondere auch magensaftresistent überzogene Tabletten oder Tabletten mit modifizierter Wirkstofffreigabe), Kapseln (Hart- und Weichgelatinekapseln), Pillen, Granulate, Suppositorien, Ovula, Salben Cremes, Gele, Pflaster, TTS oder auch als Emulsionen, Suspensionen, Lösungen oder rekonstituierbare Pulver (auch zur parenteralen Applikation) vorliegen.

Die Dosierung hängt vom Alter, Zustand und Gewicht des Patienten sowie von der Applikationsart ab. In der Regel beträgt die tägliche Wirkstoffdosis 2 bis 10 mg/kg Körpergewicht bei oraler Gabe, 1 bis 10 mg/kg Körpergewicht bei parenteraler Gabe und 0.1 bis 0.5 mg/kg Körpergewicht bei topischer Gabe.

Bei der Formulierung verwendete Exzipienten können Füllstoffe (Träger), Vehikel, Verdünnungsmittel, Lösungsmittel darunter einwertige Alkohole, wie Ethanol, Isopropanol und mehrwertige Alkohole wie Glykole, sowie Speiseöle wie Sojaöl, Kokosnussöl, Olivenöl, Distelöl, Baumwollsamenöl, ölige Ester wie Ethyloleat, Isopropylmyristat; Bindemittel, Hilfsstoffe, Löslichkeitsvermittler, Eindickmittel, Stabilisierungsmittel, Fließregulierungsmittel, Schmiermittel, Pufferstoffe, Emulgatoren, Netzmitteln, Dispergiermitteln, Süßungsmittel, Farbstoffe, Aromastoffe, Hüllstoffe, Konservierungsmittel, Antioxidantien, Tablettensprengmittel, Weichmacher, Sorptionsmittel und/oder Retardierungsmittel wie Calciumphosphat, Magnesiumstearat, Talk, Monosaccharide, Disaccharide, Stärke, Gelatine, Cellulose, Methylcellulose, Natriumcarboxymethylcellulose, Dextrose, Hydroxypropyl-β-Cyclodextrin, Polyvinylpyrrolidin, niederschmelzende Wachse und lonenaustauscherharze, umfassen.

Die so erhaltenen Applikationsformen enthalten den Wirkstoff in einer Menge von 1 bis 99Gew.-%, vorzugsweise in einer Menge von 20 bis 99 Gew.%.

Die nachstehenden Beispiele belegen die Wirksamkeit der erfindungsgemäßen Verwendung.

### Beispiel 1

Dobesilat-Calcium wurde in einer Dosis von 1400 mg je adultes Pferd jeweils zweimal täglich mit dem Futter verabreicht (entspricht 4mg/kg KGW/d). Die Therapie wird während 4 Monaten durchgeführt. Der Therapieverlauf wird nach einem Monat, nach zwei Monaten und nach 4 Monaten sonographisch dokumentiert. Während der Therapie werden die Pferde im Schritt bewegt.

Der Therapieverlauf zeigt sonographisch stets folgende Merkmale: Das in Folge von zerrisenen Sehnenfibrillenund Bluergüssen geschädigte Sehnengewebe wird abgebaut und durch ausgerichtete kollagene Fasern ersetzt, d.h. die zu Therapiebeginn hypoechogenen Sehnenabschnitte zeigen am Ende der Behandlung im Ultraschallbild eine physiologische Struktur und Dichte der Sehne.

## Patentansprüche

1. Verwendung von Dobesilat-Calcium (2,5-Dihydroxybenzosulfonat) zur Herstellung eines Arzneimittels zur Behandlung und/oder Prophylaxe von Sehnenerkrankungen.

2. Verwendung nach Anspruch 1, wobei die Sehnenerkrankung eine Tendinitis ist.

3. Verwendung nach Anspruch 1 oder 2, wobei die Behandlung und Prophylaxe an Menschen oder Säugetieren vorzunehmen ist.

4. Verwendung nach Anspruch 3, wobei die Behandlung und/oder Prophylaxe am Pferd vorzunehmen ist.

5. Verwendung nach Anspruch 4 zur Behandlung einer Tendinitis der oberflächlichen Beugesehne beim Pferd.

6. Verwendung nach einem der Ansprüche 1 - 5, wobei das Arzneimittel weiterhin thrombozytenaggregationshemmende Wirkstoffe enthält.

7. Verwendung nach Anspruch 6, wobei der thrombozytenaggregationshemmende Wirkstoff ausgewählt ist aus Acetylsalicylsäure (ASS) und/oder Benzopyronverbindungen.

8. Verwendung nach Anspruch 7, wobei die Benzopyronverbindungen ausgewählt sind aus Cumarin, Hydroxycumarin, Rutin, Monoxerutin, Troxerutin und Diosnin.

9. Verwendung nach einem der Ansprüche 1 - 8, wobei das Arzneimittel weiter einen oder mehrere pharmazeutisch verträgliche Exzipienten enthält.

10. Verwendung nach einem der Ansprüche 1 - 9, wobei das Arzneimittel zur oralen, rektalen, topischen oder parenteralen Applikation ist.

11. Verwendung nach Anspruch 10, wobei das Arzneimittel zur oralen Applikation ist.

## Claims

1. Use of calcium dobesilate (2,5-dihydroxybenzosulfonate) for the preparation of a pharmaceutical composition for the treatment and/or prophylaxis of tendon diseases.

2. Use according to claim 1, wherein the tendon disease is tendinitis.

3. Use according to claim 1 or 2, wherein the treatment and prophylaxis is carried out in humans or mammals.

4. Use according to claim 3, wherein the treatment and/or prophylaxis is carried out in horses.

5. Use according to claim 4 for the treatment of tendinitis of the superficial digital flexor tendon of the horse.

6. Use according to any one of claims 1 to 5, wherein the pharmaceutical composition further contains platelet aggregation inhibitors.

7. Use according to claim 6, wherein the platelet aggregation inhibitor is selected from acetylsalicylic acid (ASA) and/or benzopyrone compounds.

8. Use according to claim 7, wherein the benzopyrone compounds are selected from coumarin, hydroxycoumarin, rutin, monoxerutin, troxerutin and diosnin.

9. Use according to any one of claims 1 to 8, wherein the pharmaceutical composition further contains one or more pharmaceutically acceptable excipients.

10. Use according to any one of claims 1 to 9, wherein the pharmaceutical composition is for oral, rectal, topical or parenteral application.

11. Use according to claim 10, wherein the pharmaceutical composition is for oral application.

## Revendications

1. Utilisation de dobésilate de calcium (2,5-dihydroxybenzosulfonate) pour la préparation d'une composition pharmaceutique pour le traitement et/ ou la prophylaxie d'affections du tendon.

2. Utilisation selon la revendication 1, dans laquelle l'affection du tendon est la tendinite.

3. Utilisation selon la revendication 1 ou 2, dans laquelle le traitement et la prophylaxie sont réalisés chez des humains ou chez des mammifères.

4. Utilisation selon la revendication 3, dans laquelle le traitement et/ou la prophylaxie est réalisé chez des chevaux.

5. Utilisation selon la revendication 4 pour le traitement des tendinites du tendon fléchisseur digital superficiel du cheval.

6. Utilisation selon l'une quelconque des revendications 1 à 5, dans laquelle la composition pharmaceutique contient en outre des inhibiteurs de l'agrégation des plaquettes.

7. Utilisation selon la revendication 6, dans laquelle l'inhibiteur de l'agrégation des plaquettes est choisi parmi l'acide acétylsalicylique (ASA) et/ou les composés benzopyrones.

8. Utilisation selon la revendication 7, dans laquelle les composés benzopyrones sont choisis parmi la coumarine, l'hydroxycoumarine, la rutine, la monoxérutine, la troxérutine et la diosnine.

9. Utilisation selon l'une quelconque des revendications 1 à 8, dans laquelle la composition pharmaceutique contient en outre un ou plusieurs excipients pharmaceutiquement acceptables.

10. Utilisation selon l'une quelconque des revendications 1 à 9, dans laquelle la composition pharmaceutique est destinée à une administration orale, rectale, topique ou parentérale.

11. Utilisation selon la revendication 10, dans laquelle la composition pharmaceutique est destinée à une administration orale.
